# EUROPEAN PATENT SPECIFICATION

(11) **EP 2 210 248 B1**
(45) Date of publication and mention of the grant of the patent: **20.04.2016**
(21) Application number: 08850333.9
(22) Date of filing: 30.10.2008
(51) Int. Cl.: G09B 23/28, A61F 2/06

(54) **INTRALUMINAL BYPASS PROSTHESIS**
INTRALUMINALE BYPASS-PROTHESE
PROTHÈSE DE DÉRIVATION INTRALUMINALE

(30) Priority: 13.11.2007 US 987660 P
(43) Date of publication of application: 28.07.2010
(73) Proprietor: Cook Medical Technologies LLC, Bloomington, IN 47404 (US)
(72) Inventor: MEAD, Jason, A., Plainfield, IN 46168 (US)
(74) Representative: Jehan, Robert
(86) International application number: PCT/US2008/012321
(87) International publication number: WO 2009/064353

(56) References cited:
- WO-A-00/57817
- WO-A-95/14442
- WO-A-98/08456
- US-A1- 2004 215 220

## Description

### Technical Field

This invention relates to an intraluminal bypass prosthesis, and to a prosthesis delivery and deployment kit.

### Background of the Invention

The functional vessels of animal bodies, such as blood vessels, respiratory vessels, gastrointestinal vessels, and ducts, occasionally become damaged or diseased. For example, blood vessels such as the aorta can weaken and expand, forming an aneurysm. If left untreated, haemodynamic forces can cause the aneurysm to rupture, resulting in internal bleeding, and often death.

Other conditions that affect the functional vessels of animal bodies include occlusive diseases. For example, blood vessels can harden and narrow, or become partially occluded through thrombosis, resulting in a decrease in the function of the vessel. If the condition is left untreated, the vessel can become completely occluded, resulting in a complete loss of function, and in death. Alternatively, if a piece of thrombosis becomes dislodged, it can travel through the vessel and may cause an embolism.

Various techniques are currently used to repair damaged and diseased vessels. These include surgical techniques and intraluminal techniques. In general, surgical techniques involve repairing the diseased vessel by direct exposure, for example by opening and resecting, or physically removing, the diseased portion of the vessel. Surgical techniques are typically highly invasive and involve cutting into the body to access directly the diseased or damaged vessel. Often, in order to gain access to the damaged vessel, one or more organs may need to be moved or removed from the body. Once the procedure is complete, a patient may spend days in intensive care, and complete recovery may take months.

Intraluminal techniques, on the other hand, are generally less invasive and do not require direct access to the diseased vessel. Rather, an expandable prosthesis may be provided and introduced into the vessel, typically through a remote access site, such as a branch vessel. For example, in the case of an abdominal aortic aneurysm, an expandable prosthesis may be introduced via a catheter through a femoral or brachial artery. The prosthesis is then delivered to the repair site, whereupon it is expanded into contact with the aorta. The prosthesis relines the aorta and excludes blood flow to the aneurysm.

Various intraluminal prostheses, delivery and deployment devices, and methods of delivering and deploying intraluminal prostheses have been proposed for repairing damaged or diseased body lumens. For example, PCT Publication No. WO 98/53761, entitled "A Prosthesis and a Method and Means of Deploying a Prosthesis," U.S. Patent Nos. 6,524,335, entitled "Endoluminal Aortic Stents," and 7,160,318, entitled "Modular Stent Graft Assembly and Use Thereof," and U.S. Patent Application Publication Nos. 2003/0199967 A1, entitled "Bifurcated/Branch Vessel Prosthesis," 2004/0082990 A1, entitled "Composite Prosthesis," 2004/0106978 A1, entitled "Thoracic Aortic Aneurysm Stent Graft," 2004/0230287 A1, entitled "Branch Stent Graft Deployment and Method," 2005/0131519 A1, entitled "Composite Stent Graft," and 2006/0247761 A1, entitled "Branched Vessel Endoluminal Device with Fenestration" disclose exemplary devices and methods.

US 2004/0215220 discloses an anastomotic stent, apparatus and methods of use thereof.

In many cases, intraluminal techniques are preferred over more invasive surgical techniques. However, not all patients may be candidates for intraluminal repair using presently known devices and techniques. For example, severe vessel tortuosity and disease may make intraluminal repair difficult and each is a variable that may exclude a patient from consideration for intraluminal repair. Moreover, even when a patient is a candidate for intraluminal repair, known intraluminal devices may not be suitable or sufficient for the patient, and surgical techniques may be required.

For example, in the case where an abdominal aortic aneurysm extends into an iliac artery, it may not be possible completely to repair the aneurysm using a traditional intraluminal prosthesis. In these cases, a tubular intraluminal device may be provided to repair the aneurysm in the aorta. Such a device may typically extend from the aorta into a single iliac artery and exclude blood flow to the diseased iliac. Next, a surgical graft may be sutured between the femoral arteries to restore fluid communication to the diseased leg. This surgical procedure is termed a femoral-femoral ("fem-fem") bypass. Other examples of surgical bypasses include, but are not limited to, femoral-popliteal bypass grafting, coronary artery bypass grafting, and haemodialysis grafting. The ability to perform each of these procedures using intraluminal devices would constitute a major improvement in intraluminal therapies.

In general, the process of delivering and deploying an intraluminal prosthesis is complex and requires extensive experience and skill. Each patient's anatomy is unique and presents different challenges. Common anatomical variations include vessel size, spatial and angular relationships between communicating vessels, and vessel tortuosity. The physician has various tools for determining a patient's anatomical landscape prior to, and during, a procedure. For example, medical imaging techniques such as fluoroscopy, ultrasound, computed tomography (CT) and magnetic resonance imaging (MRI) are available and can be used to study a patient's anatomy. Based on the images, a physician may be able to predict and prepare for procedural challenges. While these tools are typically adequate, it would be beneficial to provide the physician the ability to practise a procedure prior to, or even during the actual procedure.

### Summary of the Invention

According to an aspect of the present invention there is provided an intraluminal bypass prosthesis as specified in claim 1.

An intraluminal bypass prosthesis is provided and comprises a first prosthetic module, a second prosthetic module, and a third prosthetic module, where the first prosthetic module has a first end, a second end, and a first fenestration disposed between the first and second ends; the second prosthetic module has a first end, a second end, and a second fenestration disposed between the first and second ends; and the third prosthetic module has a first end that is sealingly engageable with the first module within the first fenestration, a second end that is sealingly engageable with the second module within the second fenestration, and a lumen between the first and second ends of the third prosthetic module for providing fluid communication between the first and second prosthetic modules.

One or more of the prosthetic modules may be a stent graft and comprise one or more stents. In one example, the first and third prosthetic modules may be stent grafts. In another example, each of the prosthetic modules may be stent grafts. In another example, the third prosthetic module may comprise a balloon-expandable stent disposed at the first and second ends and one or more self-expanding stents disposed intermediate the first and second ends. At least one of the fenestrations in the first and second prosthetic modules may comprise a branch lumen extending from the prosthetic module.

The third prosthetic module comprises a structure that allows it sealingly to engage the first and second prostheses. Both of the first and second ends of the third prosthetic module comprise an anchor. In some examples, one end of the second module is at least partially occluded to prevent fluid flow through that end.

A prosthesis may be provided, as described above, where the first prosthetic module is placed in a first vessel segment and the second prosthetic module is placed in a second vessel segment. The third prosthetic module is placed between the first and second prosthetic modules so that the first end of the third module sealingly engages the first module within the first fenestration, and the second end of the third module sealingly engages the second module within the second fenestration.

The first vessel segment and the second vessel segment may comprise portions of the same vessel. Alternatively, in an example not forming part of the invention, the first and second vessel segments may comprise portions of different vessels, for example, an artery and a vein, left and right femoral arteries, or the left common carotid and left subclavian arteries.

A prosthesis for occluding a vessel is disclosed and comprises an intraluminal graft having a first end, a second end, and a lumen disposed between the first and second ends. The graft is at least partially occluded to prevent fluid flow through the first end of the graft. For example, the graft may comprise a graft material that traverses and closes the lumen at the first end of the graft. The prosthesis further comprises a fenestration disposed between the first and second ends of the graft. In some examples, the fenestration is the graft inlet and the second end is the graft outlet. In other examples, the second end is the graft inlet and the fenestration is the graft outlet.

The prosthesis, as described above, may further comprise a second intraluminal graft having a first end, a second end, and a lumen disposed between the first and second ends. The second graft may be sealingly engageable with the first graft to provide fluid communication between the first and second grafts. For example, the first end of the second graft may sealingly engage the first graft within the fenestration to provide fluid communication between the first and second grafts.

The prosthesis, as described above, may further comprise a third intraluminal graft having a first end, a second end, and a lumen disposed between the first and second ends. The prosthesis may comprise a second graft and the third graft may be sealingly engageable with the second graft to provide fluid communication between the first and third grafts. In some examples, the third graft may comprise a second fenestration disposed between the first and second ends of the third graft. The second end of the second graft may sealingly engage the third graft within the second fenestration to provide fluid communication between the first and third grafts. In some examples, the first end of the third graft is at least partially occluded to prevent fluid flow therethrough.

A prosthesis delivery and deployment kit is disclosed as an example not forming part of the invention as claimed and comprises a deployment model and a plurality of intraluminal prostheses. In some examples, the deployment model comprises one or more vessels corresponding generally with a patient's vessels. The kit may comprise a first intraluminal prosthesis for delivery and deployment within the patient, and a second intraluminal prosthesis, substantially identical to the first intraluminal prosthesis, for delivery and deployment within the deployment model. Such a kit may be advantageous, for example, where the physician wishes to practice a patient's procedure prior to, or even during, the actual procedure.

A method of treating a damaged or diseased vessel is described, but does not form part of the invention as claimed, and comprises the steps of delivering and deploying a first prosthetic module into a first vessel segment, the first prosthetic module comprising a first end, a second end, and a first fenestration disposed between the first and second ends; delivering and deploying a second prosthetic module into a second vessel segment, the second prosthetic module comprising a first end, a second end, and a second fenestration disposed between the first and second ends; creating a channel between the first and second prosthetic modules; and delivering and deploying a third prosthetic module within the channel. The deploying step may comprise sealingly engaging a first end of the third prosthetic module with the first module within the first fenestration, and sealingly engaging a second end of the third prosthetic module with the second module within the second fenestration, to provide fluid communication between the first and second prosthetic modules.

### Brief Description of the Drawing

Preferred embodiments of the present invention are now described by way of example only, and with reference to the accompanying drawings, in which:
FIGS 1A-1C depict various examples of damaged or diseased body vessels;
FIGS 2A-2F depict various examples of prosthetic modules;
FIG 3 depicts an example of an intraluminal graft that may be used to occlude a vessel;
FIG 4 depicts an intraluminal bypass prosthesis for repairing a damaged or diseased body lumen;
FIG 5 depicts an embodiment of the intraluminal bypass prosthesis of the current invention for repairing an occluded vessel;
FIG 6 is a perspective view of an exemplary delivery and deployment device;
FIG 7 is a cross-sectional view of the device of FIG 6;
FIG 8 is a perspective view of selected segments of a delivery and deployment device including a partially-deployed prosthesis;
FIG 9 depicts another exemplary delivery and deployment device;
FIG 10 depicts a distal end of the device of FIG 9; and
FIGS 11A-11F depict various stages of an exemplary delivery and deployment method for an intraluminal bypass prosthesis.

### Detailed Description

Throughout the specification and in the appended claims, when referring to a prosthesis, or to a structure or component of a prosthesis, the terms "distal" and "distally" shall denote a position, direction, or orientation that is generally downstream in the direction of fluid flow. Accordingly, the terms "proximal" and "proximally" shall denote a position, direction, or orientation that is generally upstream in the direction of fluid flow. When referring to a delivery and deployment system, or to a structure or component of a delivery and deployment system, the terms "distal" and "distally" shall denote a position, direction, or orientation that is generally toward the patient and the terms "proximal" and "proximally" shall denote a position, direction, or orientation that is generally away from the patient.

The term "vessel" refers to a tube or canal in which fluid may be contained and conveyed or circulated. A body vessel (as opposed to a prosthetic vessel) is a vessel that naturally exists, or is naturally formed in the body. Examples of body vessels include, but are not limited to, blood vessels such as the aorta and the femoral artery, the esophagus, the trachea, the ureter, the bile duct, etc. Examples of prosthetic vessels include grafts and stent grafts.

The term "lumen" shall mean a cavity or channel within a tube or a tubular body, such as vessel. The term "intraluminal" means within a lumen, and can refer to objects that are found or that can be placed within a lumen, or methods or processes that occur within a lumen. An "intraluminal prosthesis" is thus a prosthesis that is found or that can be placed within a lumen.

The term "module" shall mean a fully-functional unitary device that may be used either independently, or in combination with one or more other modules.

The term "prosthesis" shall mean any device, object, or structure that supports, repairs, or replaces, or is configured to support, repair, or replace, a body part or a function of that body part and includes any device that enhances or adds functionality to a physiological system. Examples of prostheses include, but are not limited to, stents, grafts, stent grafts, valves, and vena cava filters.

The term "stent" shall mean any device or structure that provides or is configured to provide rigidity, expansion force, or support to a body part, for example, a diseased, damaged, or otherwise compromised body lumen.

The term "expandable" shall mean capable of being expanded. An expandable stent is a stent that is capable of being expanded, whether by virtue of its own resilience, upon the application of an external force, or by a combination of both. Expandable stents include both self-expanding and balloon-expandable devices. Self-expanding stents may be made, for example, of stainless steel, materials with elastic memory properties, such as NITINOL, or any other suitable material. Exemplary self-expanding stents include Z-STENTS® and Zilver® stents, which are each available from Cook Incorporated, Bloomington, Indiana, USA. Balloon-expandable stents may be made, for example, of stainless steel (typically 316LSS, CoCr, etc.). Hybrid stents may be provided by combining one or more self-expanding stents or stent portions with one or more balloon-expandable stents or stent portions.

The term "graft" describes an object, device, or structure that is joined or that is capable of being joined to a body part to enhance, repair, or replace a portion or a function of that body part. Grafts that can be used to repair body vessels include, for example, films, coatings, or sheets of material that are formed or adapted to conform to the body vessel that is being enhanced, repaired, or replaced. A stent may be attached to a graft to form a "stent graft."

Biocompatible fabrics, non-woven materials and porous sheets may be used as the graft material. The graft material is preferably a woven polyester having a twill weave and a porosity of about 350 ml/min/cm² (available from VASCUTEK® Ltd., Renfrewshire, Scotland, UK). The graft material may also be other polyester fabrics, polytetrafluoroethylene (PTFE), expanded PTFE, and other synthetic materials.

The graft material may include extracellular matrix materials. The "extracellular matrix" is a collagen-rich substance that is found in between cells in animal tissue and serves as a structural element in tissues. It is typically a complex mixture of polysaccharides and proteins secreted by cells. The extracellular matrix can be isolated and treated in a variety of ways. Following isolation and treatment, it is referred to as an "extracellular matrix material," or ECMM. ECMMs may be isolated from submucosa (including small intestine submucosa), stomach submucosa, urinary bladder submucosa, tissue mucosa, renal capsule, dura mater, liver basement membrane, pericardium or other tissues.

Purified tela submucosa, a preferred type of ECMM, has been previously described in U.S. Patent Nos. 6,206,931; 6,358,284 and 6,666,892 as a biocompatible, non-thrombogenic material that enhances the repair of damaged or diseased host tissues. Purified submucosa extracted from the small intestine ("small intestine submucosa" or "SIS") is a more preferred type of ECMM for use in this invention. Another type of ECMM, isolated from liver basement membrane, is described in U.S. Patent No. 6,379,710, ECMM may also be isolated from pericardium, as described in U.S. Patent No. 4,502,159. Other examples of ECMMs are stomach submucosa, liver basement membrane, urinary bladder submucosa, tissue mucosa and dura mater. SIS can be made in the fashion described in U.S. Patent No. 4,902,508 to Badylak et al.; U.S. Patent No. 5,733,337 to Carr; U.S. Patent No. 6,206,931 to Cook et al.; U.S. Patent No. 6,358,284 to Feamot et al.; 17 Nature Biotechnology 1083 (Nov. 1999); and PCT Publication No. WO 98/22158 of May 28, 1998 to Cook et al.*,* which is the published application of PCT/US97/14855. It is also preferable.that the material is non-porous so that it does not leak or sweat under physiologic forces.

Biocompatible polyurethanes may also be employed as graft materials. One example of a biocompatible polyurethane is THORALON (THORATEC, Pleasanton CA), as described in U.S. Patent Nos. 6,939,377 and 4,675,361. THORALON is a polyurethane base polymer (referred to as BPS-215) blended with a siloxane-containing surface-modifying additive (referred to as SMA-300). The concentration of the surface modifying additive may be in the range of 0.5% to 5% by weight of the base polymer.

The SMA-300 component (THORATEC) is a polyurethane comprising polydimethylsiloxane as a soft segment and the reaction product of diphenylmethane diisocyanate (MDI) and 1,4-butanediol as a hard segment. A process for synthesizing SMA-300 is described, for example, in U.S. Patent Nos. 4,861,830 and 4,675,361.

The BPS-215 component (THORATEC) is a segmented polyetherurethane urea containing a soft segment and a hard segment. The soft segment is made of polytetramethylene oxide (PTMO), and the hard segment is made from the reaction of 4,4'-diphenylmethane diisocyanate (MDI) and ethylene diamine (ED).

THORALON can be manipulated to provide either porous or non-porous THORALON. Porous THORALON can be formed by mixing the polyetherurethane urea (BPS-215), the surface modifying additive (SMA-300) and a particulate substance in a solvent. The particulate may be any of a variety of different particulates or pore forming agents, including inorganic salts. Preferably the particulate is insoluble in the solvent. The solvent may include dimethyl formamide (DMF), tetrahydrofuran (THF), dimethyacetamide (DMAC), dimethyl sulfoxide (DMSO) or mixtures thereof. The composition can contain from about 5 wt% to about 40 wt% polymer, and different levels of polymer within the range can be used to fine tune the viscosity needed for a given process. The composition can contain less than 5 wt% polymer for some spray application embodiments. The particulates can be mixed into the composition. For example, the mixing can be performed with a spinning blade mixer for about an hour under ambient pressure and in a temperature range of about 18°C to about 27°C. The entire composition can be cast as a sheet, or coated onto an article such as a mandrel or a mould. In one example, the composition can be dried to remove the solvent, and then the dried material can be soaked in distilled water to dissolve the particulates and leave pores in the material. In another example, the composition can be coagulated in a bath of distilled water. Since the polymer is insoluble in the water, it will rapidly solidify, trapping some or all of the particulates. The particulates can then dissolve from the polymer, leaving pores in the material. It may be desirable to use warm water for the extraction, for example, water at a temperature of about 60°C. The resulting pore diameter can also be substantially equal to the diameter of the salt grains.

The porous polymeric sheet can have a void-to-volume ratio from about 0.40 to about 0.90. Preferably the void-to-volume ratio is from about 0.65 to about 0.80. The resulting void-to-volume ratio can be substantially equal to the ratio of salt volume to the volume of the polymer plus the salt. Void-to-volume ratio is defined as the volume of the pores divided by the total volume of the polymeric layer including the volume of the pores. The void-to-volume ratio can be measured using the protocol described in AAMI (Association for the Advancement of Medical Instrumentation) VP20-1994, Cardiovascular Implants -- Vascular Prosthesis section 8.2.1.2, Method for Gravimetric Determination of Porosity. The pores in the polymer can have an average pore diameter from about 1 micron to about 400 microns. Preferably, the average pore diameter is from about 1 micron to about 100 microns; more preferably, it is from about 1 micron to about 10 microns. The average pore diameter is measured based on images from a scanning electron microscope (SEM). Formation of porous THORALON is described, for example, in U.S. Patent No. 6,752,826 and US. Patent Application Publication No. 2003/0149471 A1.

Non-porous THORALON can be formed by mixing the polyetherurethane urea (BPS-21 5) and the surface modifying additive (SMA-300) in a solvent, such as dimethyl formamide (DMF), tetrahydrofuran (THF), dimethyacetamide (DMAC) or dimethyl sulfoxide (DMSO). The composition can contain from about 5 wt% to about 40 wt% polymer, and different levels of polymer within the range can be used to fine tune the viscosity needed for a given process. The composition can contain less than 5 wt% polymer for some spray application embodiments. The entire composition can be cast as a sheet, or coated onto an article such as a mandrel or a mold. In one example, the composition can be dried to remove the solvent.

THORALON has been used in certain vascular applications and is characterized by thromboresistance, high tensile strength, low water absorption, low critical surface tension, and good flex life. THORALON is believed to be biostable and useful *in vivo* in long term blood contacting applications requiring biostability and leak resistance. Because of its flexibility, THORALON is useful in larger vessels, such as the abdominal aorta, where elasticity and compliance is beneficial.

A variety of other biocompatible polyurethanes may also be employed. These include polyurethanes that preferably include a soft segment and include a hard segment formed from a diisocyanate and diamine. For example, polyurethane with soft segments such as PTMO, polyethylene oxide, polypropylene oxide, polycarbonate, polyolefin, polysiloxane (i.e. polydimethylsiloxane), and other polyether soft segments made from higher homologous series of diols may be used. Mixtures of any of the soft segments may also be used. The soft segments also may have either alcohol end groups or amine end groups. The molecular weight of the soft segments may vary from about 500 to about 5,000 g/mole.

The diisocyanate used as a component of the hard segment may be represented by the formula OCN-R-NCO, where -R- may be aliphatic, aromatic, cycloaliphatic or a mixture of aliphatic and aromatic moieties. Examples of diisocyanates include MDI, tetramethylene diisocyanate, hexamethylene diisocyanate, trimethyhexamethylene diisocyanate, tetramethylxylylene diisocyanate, 4,4'-dicyclohexylmethane diisocyanate, dimer acid diisocyanate, isophorone diisocyanate, metaxylene diisocyanate, diethylbenzene diisocyanate, decamethylene 1,10 diisocyanate, cyclohexylene 1,2-diisocyanate, 2,4-toluene diisocyanate, 2,6-toluene diisocyanate, xylene diisocyanate, m-phenylene diisocyanate, hexahydrotolylene diisocyanate (and isomers), naphthylene-1,5-diisocyanate, 1-methoxyphenyl 2,4-diisocyanate, 4,4'-biphenylene diisocyanate, 3,3'-dimethoxy-4,4'-biphenyl diisocyanate and mixtures thereof.

The diamine used as a component of the hard segment includes aliphatic amines, aromatic amines and amines containing both aliphatic and aromatic moieties. For example, diamines include ethylene diamine, propane diamines, butanediamines, hexanediamines, pentane diamines, heptane diamines, octane diamines, m-xylylene diamine, 1,4-cyclohexane diamine, 2-methypentamethylene diamine, 4,4'-methylene dianiline and mixtures thereof. The amines may also contain oxygen and/or halogen atoms in their structures.

Other applicable biocompatible polyurethanes include those using a polyol as a component of the hard segment. Polyols may be aliphatic, aromatic, cycloaliphatic or may contain a mixture of aliphatic and aromatic moieties. For example, the polyol may be ethylene glycol, diethylene glycol, triethylene glycol, 1,4-butanediol, 1,6-hexanediol, 1,8-octanediol, propylene glycols, 2,3-butylene glycol, dipropylene glycol, dibutylene glycol, glycerol, or mixtures thereof.

Biocompatible polyurethanes modified with cationic, anionic and aliphatic side chains may also be used, as in U.S. Patent No. 5,017,664.

Other biocompatible polyurethanes include: segmented polyurethanes, such as BIOSPAN; polycarbonate urethanes, such as BIONATE; and polyetherurethanes, such as ELASTHANE; (all available from POLYMER TECHNOLOGY GROUP, Berkeley, California, USA).

Other biocompatible polyurethanes include polyurethanes having siloxane segments, also referred to as a siloxane-polyurethane. Examples of polyurethanes containing siloxane segments include polyether siloxanepolyurethanes, polycarbonate siloxane-polyurethanes, and siloxanepolyurethane ureas. Specifically, examples of siloxane-polyurethane include polymers such as ELAST-EON 2 and ELAST-EON 3 (AORTECH BIOMATERIALS, Victoria, Australia); polytetramethyleneoxide (PTMO) and polydimethylsiloxane (PDMS) polyether-based aromatic siloxanepolyurethanes such as PURSIL-10, -20, and -40 TSPU; PTMO and PDMS polyether-based aliphatic siloxane-polyurethanes such as PURSIL AL-5 and AL-10 TSPU; aliphatic, hydroxy-terminated polycarbonate and PDMS polycarbonate-based siloxane-polyurethanes such as CARBOSIL-10, -20, and -40 TSPU (all available from POLYMER TECHNOLOGY GROUP). The PURSIL, PURSIL-AL, and CARBOSIL polymers are thermoplastic elastomer urethane copolymers containing siloxane in the soft segment, and the percent siloxane in the copolymer is referred to in the grade name. For example, PURSIL-10 contains 10% siloxane. These polymers are synthesized through a multi-step bulk synthesis in which PDMS is incorporated into the polymer soft segment with PTMO (PURSIL) or an aliphatic hydroxy-terminated polycarbonate (CARBOSIL). The hard segment consists of the reaction product of an aromatic diisocyanate, MDI, with a low molecular weight glycol chain extender. In the case of PURSIL-AL, the hard segment is synthesized from an aliphatic diisocyanate. The polymer chains are then terminated with a siloxane or other surface modifying end group. Siloxane-polyurethanes typically have a relatively low glass transition temperature, which provides for polymeric materials having increased flexibility relative to many conventional materials. In addition, the siloxane-polyurethane can exhibit high hydrolytic and oxidative stability, including improved resistance to environmental stress cracking. Examples of siloxane-polyurethanes are disclosed in U.S. Patent Application Publication No. 2002/0187288 A1

In addition, any of these biocompatible polyurethanes may be end-capped with surface active end groups, such as, for example, polydimethylsiloxane, fluoropolymers, polyolefin, polyethylene oxide or other suitable groups. See, for example, the surface active end groups disclosed in U.S. Patent No. 5,589,563.

### INTRALUMINAL BYPASS PROSTHESIS

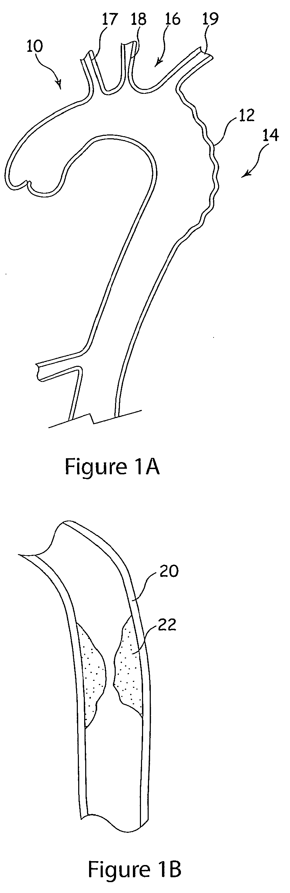

FIG 1A shows a schematic view of the aorta 10 of a patient with an aneurysm 12 that has formed in the descending aorta 14 and the aortic arch 16. Several branch vessels are shown and extend from the aortic arch 16, including the innominate 17, the left common carotid 18, and the left subclavian 19 arteries. These branch vessels provide fluid communication to various locations within the body, including the arms, neck, and head. The aneurysm 12 may be repaired, for example, by surgical resection and grafting, or by intraluminal grafting. Depending on severity of disease, intraluminal grafting may require the placement of an intraluminal prosthesis (not shown) within the aortic arch 16 so that it crosses the left subclavian artery 19. Such a prosthesis may occlude flow to the subclavian artery 19. To restore fluid communication to the subclavian artery 19, a surgeon would typically open the patient's chest and suture a surgical graft, between the left common carotid and subclavian arteries 18, 19.

FIG 1B shows a cross-sectional view of a diseased vessel 20 that is partially occluded due to a build-up of thrombosis 22. The vessel 20 may be repaired, for example, by balloon angioplasty to open the vessel and/or to break up and remove the thrombosis. Additionally, or alternatively, an intraluminal device such as a stent or stent graft (not shown) may be placed to reline and restore patency to the vessel. In some cases, the vessel may be significantly diseased so that it may not be possible to place a balloon or a prosthesis at the diseased site. In such cases, the vessel would typically be repaired by surgically removing the diseased portion and replacing it with a surgical graft.

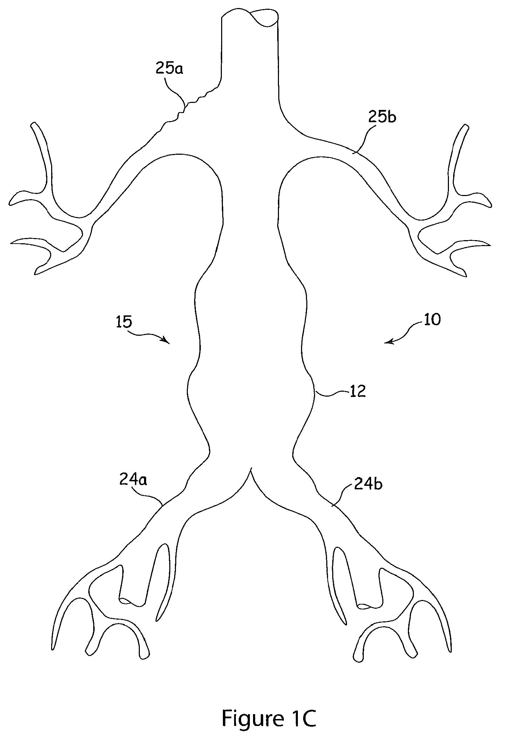

FIG 1C shows a schematic view of the aorta 10 of a patient with an aneurysm 12 that has formed in the abdominal aorta 15. Several branch vessels are shown, including the iliac arteries 24a, 24b, and the renal arteries 25a, 25b. The aneurysym 12 extends into the left renal artery 25a, as well as the right iliac artery 24b. The aneurysm 12 may be repaired, for example, by intraluminal delivery and deployment of a bifurcated graft (not shown). Alternatively, an aorto-uniiliac graft (not shown) may be provided and implanted between the aorta 15 and iliac artery 24a to direct blood flow into the iliac artery 24a and to exclude blood flow to the other iliac artery 24b. To restore fluid communication to the diseased leg, a surgeon would typically open the patient's abdomen and suture a surgical graft between the left and right femoral arteries (not shown).

Each of the examples shown in FIGS 1A-1C involves a condition that may require the implantation of a bypass graft between multiple vessel segments. Various intraluminal devices and techniques are described throughout the specification that may be used instead of, or in conjunction with, surgical devices and techniques that are typically used in these, and other like situations.

According to an embodiment of the present invention, an intraluminal bypass prosthesis may be formed using one or more intraluminal prosthetic modules. For example, a plurality of modules may be provided and assembled both within and between vessel segments to form an intraluminal, rather than a surgical, bypass.

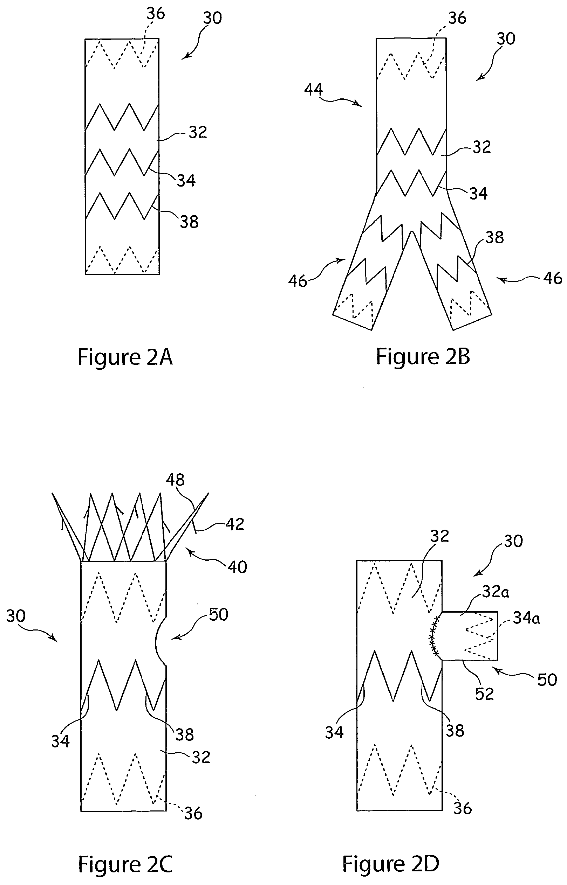

FIGS 2A-2F show examples of prosthetic modules that may be used in combination to form an intraluminal bypass prosthesis. Suitable modules that may be used with the present invention are disclosed, for example, in PCT Application No. WO 98/53761, U.S. Patent Nos. 6,524,335 and 7,160,318, and U.S. Patent Application Publication Nos. 2003/0199967 A1, 2004/0082990 A1, 2004/0106978 A1, 2004/0230287 A1, 2005/0131519 A1, and 2006/0247761 A1.

Each of the Figures shows a prosthetic module 30 comprising a graft 32 and/or one or more expandable stents 34. The stents 34 may be disposed internally and/or externally of the graft 32 and may be attached to the graft material, for example, by sutures, adhesive, and/or by incorporation of the stents with the graft material. Internal stents are desireable because they may provide a smooth external prosthesis surface that may help seal the prosthesis against the adjoining vessel. On the other hand, external stents are desireable because they may provide a smooth internal prosthesis surface and may limit or prevent flow turbulence within the prosthesis. In some of the figures, the module comprises an anchor 40 that is configured to attach the prosthesis to the vessel. An anchor in the current invention includes one or more stents and/or one or more hooks and/or barbs 42.

FIG 2A shows a generally tubular prosthetic module 30 comprising internal stents 36 disposed at the graft ends and external stents 38 disposed intermediate the ends of the graft. The module comprises a single lumen extending between the ends of the module.

FIG 2B shows a bifurcated prosthetic module 30 comprising a main portion 44 and two extension portions 46 extending from the main portion 44. In this example, the extension portions 46 are similarly sized and configured with respect to one another. In other examples, the extension portions 46 may be dissimilarly sized and configured with respect to one another. For example, the extension portions 46 may have different diameters, lengths, and/or orientations with respect to each other and with respect to the main portion 44. The module shown in FIG 2B comprises three lumens and can be used, for example, to direct flow from a single vessel into two vessels. In other examples, a bifurcated prosthesis may be provided and comprise two or more lumens.

FIG 2C shows another prosthetic module 30 comprising an anchor 40. The anchor 40 comprises a stent 48 that extends at least partially beyond an end of the graft 32. The stent 48 expands radially outwardly to engage an adjacent vessel and comprises a plurality of barbs 42 configured to engage and penetrate the vessel. The module 30 further comprises an aperture or fenestration 50 disposed between the module ends for splitting and diverting at least a portion of the flow from the modular lumen.

FIG 2D shows another prosthetic module 30 comprising a fenestration 50. In this example, the fenestration 50 comprises a prosthetic branch 52 having a branch lumen. The prosthetic branch 52 comprises a graft 32a that extends generally perpendicularly from the main body. Other examples include prosthetic branches that extend at an oblique angle from the main body and prosthetic branches that extend longitudinally along and circumferentially about the main body. The prosthetic branch 52 may comprise one or more stents 34a disposed internally (as shown in FIG 2D) and/or externally of the graft.

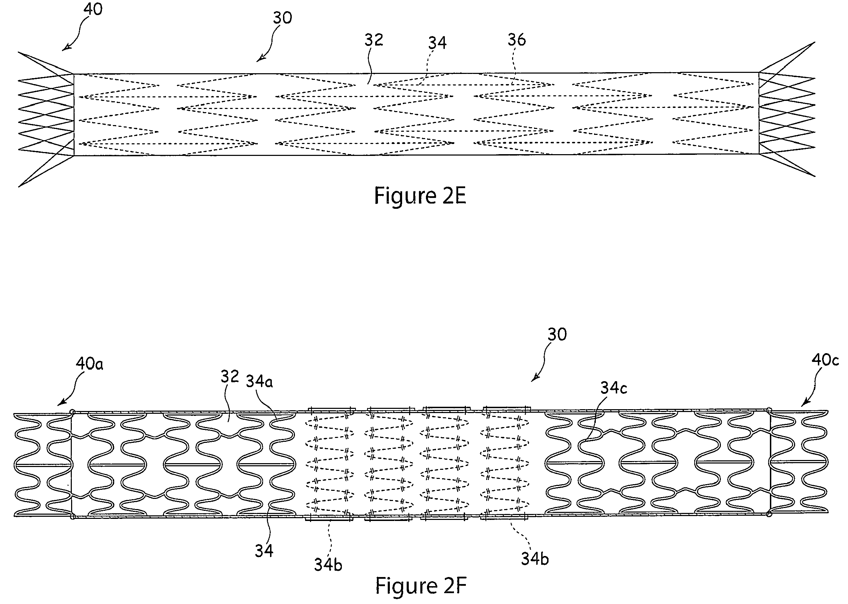

FIG 2E shows a prosthetic module 30 that comprises a graft 32 and a plurality of interconnected zigzag stents 34 extending along the length of the graft. The module may comprise, for example, a Zilver® stent, which is available from Cook Incorporated, Bloomington, Indiana, USA. In this example, each end of the module 30 comprises an anchor 40, as described above.

FIG 2F shows another prosthetic module 30 that has multiple stent portions 34a, 34b, 34c arranged along the length of the graft 32. Stent portions 34a, 34c extend beyond respective graft ends and comprise anchors 40a, 40c, as described above. Stent portion 34b is disposed along a medial portion of the graft between stent portions 34a, 34c. In one example, stent portions 34a, 34c may comprise one or more balloon-expandable stents and stent portion 34b may comprise one or more self-expanding stents, for example, Z-STENTS®.

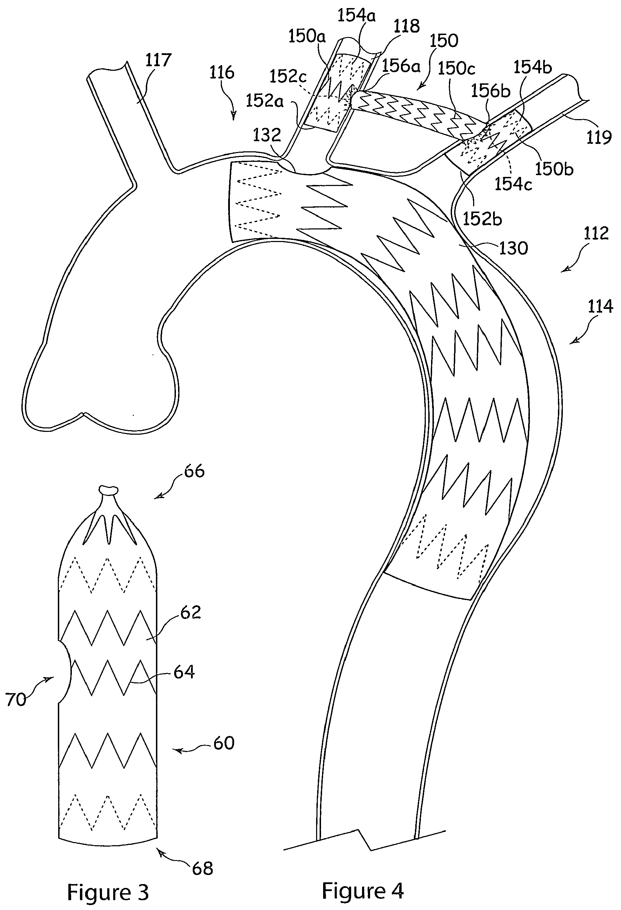

FIG 3 shows another prosthetic module 60 that may be used to repair a vessel. This module 60 comprises a graft 62 and one or more stents 64 disposed along the length of the graft 62. One end of the graft is at least partially occluded. For example, as shown in FIG 3, the graft material may traverse and close the prosthetic lumen at a first end 66 of the module 60, thereby preventing fluid flow into or out of the module through the first end. The module 60 has a second end 68 that is open, to allow fluid to flow into or out of the module. The module has a fenestration 70 disposed intermediate the first and second ends 66, 68. The fenestration 70 allows fluid to flow into or out of the module. Thus, in some examples, the module 60 may be deployed so that the second end 68 comprises the module inlet and the fenestration 70 comprises the module outlet. Alternatively, the module 60 may be deployed so that the fenestration 70 comprises the inlet and the second end 68 comprises the outlet. The fenestration 70 may comprise a prosthetic branch, as described above. Like the prosthetic modules shown in FIGS 2A-2F, the module shown in FIG 3 is combined with one or more modules.

FIG 4 shows an example of an intraluminal bypass prosthesis for repairing a damaged or diseased body lumen, for example, an aneurysm 112 of the descending thoracic aorta 114. In this example, not forming part of the invention as claimed, an intraluminal prosthesis 130 comprising one or more prosthetic modules is implanted within the distal aorta 114 and extends proximally into the aortic arch 116 and across the left common carotid 118 and left subclavian arteries 119. Prosthesis 130 extends over and occludes the opening of the left subclavian artery 119, preventing fluid communication between the aortic arch 116 and the subclavian artery. Prosthesis 130 comprises a fenestration 132 that is generally aligned with the left common carotid artery 118 to allow fluid to flow thereto.

An intraluminal bypass prosthesis 150 is provided to restore fluid communication between the aortic arch 116 and the subclavian artery 119. In the example shown in FIG 4, the bypass prosthesis 150 comprises three prosthetic modules 150a, 150b, 150c. The first prosthetic module 150a is disposed within the left common carotid artery 118 and comprises a proximal end 152a, a distal end 154a, and a fenestration 156a disposed therebetween. The second prosthetic module 150b is disposed within the left subclavian artery 119 and comprises a proximal end 152b, a distal end 154b, and a fenestration 156b disposed therebetween. The third prosthetic module 150c has a proximal end 152c in fluid communication with the carotid artery 118 and a distal end 154c in fluid communication with the subclavian artery 119. In contrast with known bypass techniques, where a single graft would be attached directly to the body vessels in end-to-side anastomoses, the third prosthetic module 150c is attached directly to the first prosthetic module 150a and to the second prosthetic module 150b. This feature has many advantages over known bypass techniques. For example, because the third prosthetic module 150c is not directly attached to a body vessel, the anastomosis between the prosthesis and the vessel may be stronger, and less prone to damage and deterioration that may be caused by sutures and other anchoring mechanisms. Moreover, the haemostatic forces within the bypass prosthesis will be directly absorbed by the prosthetic modules, rather than concentrating at the interface between the prosthesis and the body vessels.

In the example shown in FIG 4, the proximal end 152c of the third prosthetic module 150c is attached to the first module 150a via fenestration 156a, and the distal end 154c is attached to the second module 150b via fenestration 156b. Prosthetic module 150c sealingly engages first and second modules 150a, 150b and provides fluid communication therebetween.

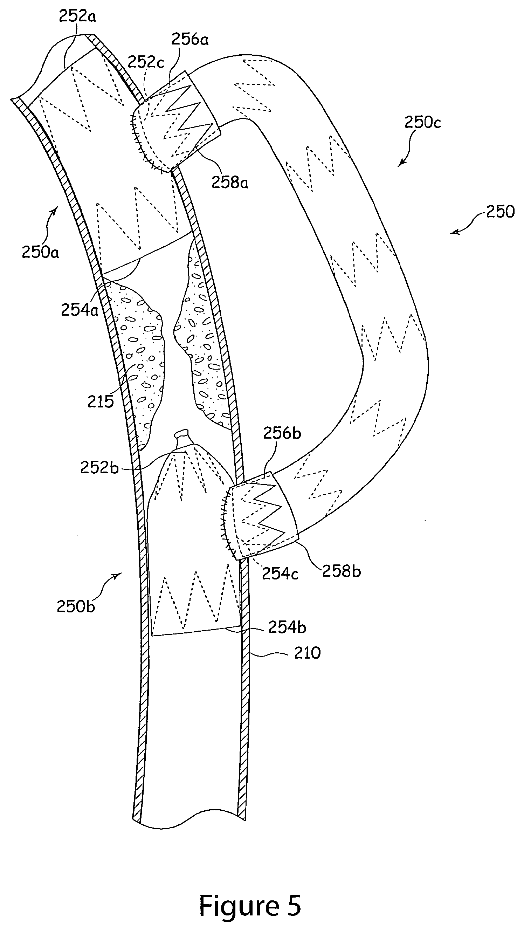

FIG 5 shows an embodiment of the intraluminal bypass prosthesis that may be used, for example, to bypass a weakened or occluded portion 215 of a vessel 210. The prosthesis 250 comprises a first prosthetic module 250a disposed proximally of the occlusion 215 within a first vessel segment, a second prosthetic module 250b disposed distally of the occlusion 215 within a second vessel segment, and a third prosthetic module 250c disposed between the first and second modules 250a, 250b. The first and second modules 250a, 250b each comprise a proximal end 252a, 252b, a distal end 254a, 254b, and a fenestration 256a, 256b disposed therebetween. The fenestrations 256a, 256b each comprise a branch fenestration including a branch lumen. The third module 250c has a proximal end 252c connected to branch fenestration 258a and a distal end 254c connected to branch fenestration 258b. The third module 250c sealingly engages the first and second modules 250a, 250b via fenestrations 256a, 256b and provides fluid communication therebetween.

In the example shown in FIG 5, the proximal end 252b of the second module 250b is at least partially occluded to prevent fluid flow between the distal end of the first module 250a and the proximal end of the second module 250b. This may reduce the pressure within the diseased portion of the vessel 215 and may contain and/or prevent embolization of any thrombosis therein. The distal end of the first module 250a may additionally, or alternatively, be occluded. In some examples, the proximal end of the second module is completely occluded. In other examples, the proximal end of the second module is partially occluded and may become completely occluded after being placed in a vessel, for example, by thrombus.

Various engagement and attachment mechanisms are contemplated for attaching prosthetic modules. For example, a prosthetic module may overlap and frictionally engage another prosthetic module. Where the engagement consists only of a friction fit between modules, slight movement between the modules may be possible. In some examples, frictional engagement may be sufficient to provide a fluid seal and to prevent the modules from separating. In the current embodiment, an anchor is provided, as shown and described above with respect to FIGS 2C, 2E, and 2F. In these examples, the anchor may be configured to mechanically interlock the modules and prevent relative movement therebetween. The anchor includes hooks and/or barbs that may engage a respective prosthetic module 150a, 150b. The anchor may be self-expanding or it may be physically expanded to a diameter that is greater than the diameter of the fenestration, thereby "locking" the modules together. Examples of suitable engagement and anchoring mechanisms and methods are described, for example, in PCT Publication No. WO 98/53761, U.S. Patent Nos. 6,524,335 and 7,160,318, and U.S. Patent Application Publication Nos. 2003/0199967 A1, 2004/0082990 A1, 2004/0106978 A1, 2004/0230287 A1, 2005/0131519 A1, and 2006/0247761 A1.

In each of the examples shown in FIGS 4 and 5, the third module is attachable to the first and second modules, and does not occlude the lumens of either module. This feature is particularly advantageous in applications, such as the one shown in FIG 4, where occlusion of the lumen of the first module 150a would prevent blood flow to vital portions of the human anatomy, such as the head and neck.

### DELIVERY AND DEPLOYMENT DEVICES

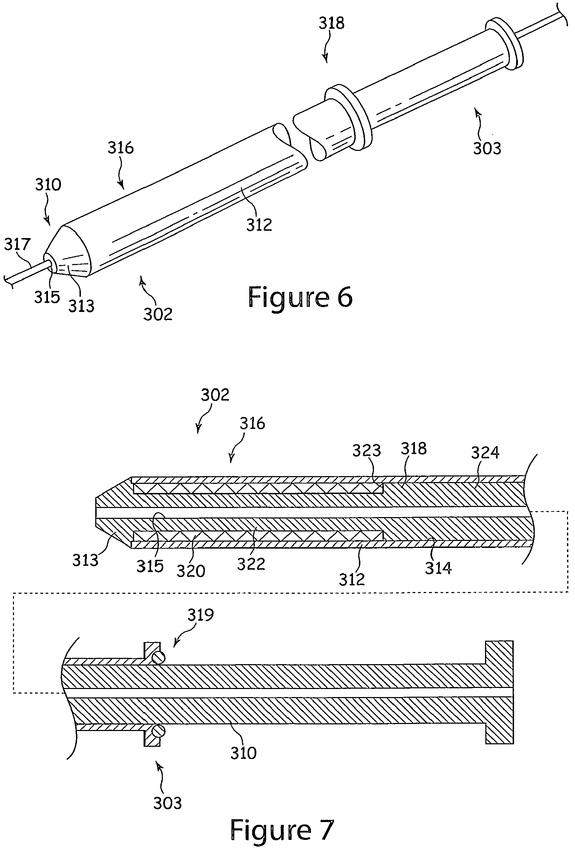

FIGS 6-10 show various exemplary devices for delivering and deploying an intraluminal prosthesis, such as a prosthetic module, in a body lumen. Said exemplary devices do not form part of the invention as claimed. The device shown in FIG 6 comprises a prosthesis delivery section 302 and an external manipulation section 303. The delivery section 302 travels through the body lumen during the procedure and delivers the prosthesis 320 (shown, for example, in FIG 7) to a desired deployment site. The external manipulation section 303 stays outside of the body during the procedure. The external manipulation section 303 can be manipulated by the operator to position and release or deploy the prosthesis 320 into the body lumen.

The delivery and deployment device comprises a delivery catheter 310 and a sheath 312. The delivery catheter 310 and the sheath 312 are configured to retain and release the expandable prosthesis 320 selectively. The delivery catheter 310 has a proximal end and a distal end. The distal end of the delivery catheter comprises a dilator head 313. The dilator head 313 is distally tapered to provide for atraumatic insertion into the body lumen (not shown). A guidewire lumen 315 extends longitudinally through the delivery catheter 310 between the proximal and distal ends. The delivery catheter 310 is configured to receive a guidewire 317 via the guidewire lumen 315 as shown in FIG 6.

The delivery catheter 310 comprises a prosthesis receiving portion 316 and a prosthesis release portion 318, as shown in FIG 6. The receiving portion 316 is disposed on a distal portion of the delivery catheter and is configured to receive the prosthesis 320 in a radially compressed configuration. As shown in FIGS 6 and 7, the receiving portion 316 may comprise a catheter tube 322 having a longitudinally uniform external diameter.

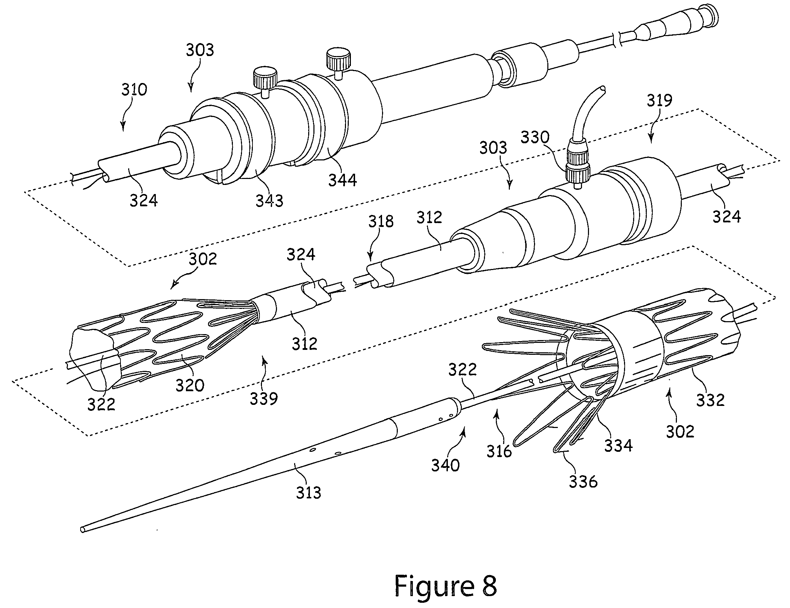

The release portion 318 of the delivery catheter 310 is disposed generally proximally of the prosthesis 320. The release portion 318 can be manipulated, along with the sheath 312, selectively to deliver and deploy the prosthesis 320 in the body lumen. As shown in FIGS 6 and 7, the release portion 318 may comprise a catheter tube 324 having a longitudinally uniform external diameter and may include a distal-facing annular abutment surface 323 at the transition between catheter tubes 322 and 324. The annular abutment surface 323 faces the proximal end of the prosthesis 320 and is configured to contact the proximal end of the prosthesis 320 during deployment, allowing the delivery catheter 310 to push the prosthesis 320 distally as the sheath 312 is pulled proximally in relation thereto. The delivery catheter 310 may comprise a single unitary structure as shown in FIG 7. Alternatively, the delivery catheter 310 may comprise a plurality of slidably interconnected catheters 322, 324 as shown in FIG 8.

The sheath 312 comprises an elongate tubular body having a proximal and distal end and a sheath lumen 314. The sheath lumen 314 has a generally constant diameter between the proximal and distal ends. The sheath 312 extends proximally from the delivery section 302 to the user manipulation section 303. The delivery catheter 310 is slidably disposed within lumen 314. The sheath 312 releasably covers and retains the prosthesis 320 in a radially reduced configuration. The dilator head 313 and the sheath 320 preferably form a generally smooth transition so as to prevent trauma to the body lumen during delivery and deployment. The distal end of the sheath 312 travels within the body lumen during a procedure. The proximal end of the sheath 312 is configured to remain outside of the body during the procedure and can be directly manipulated by the operator to deploy the prosthesis 320.

The sheath 312 may have a length, as shown in FIG 7, that is significantly greater than the length of the prosthesis 320. For example, the sheath 312 may have a length that is two or more times greater than the length of the prosthesis 320. Alternatively, the sheath 312 may have a length that is generally equal to or greater than the length of the prosthesis. The sheath 312 has a uniform internal diameter. The internal diameter of the sheath 312 is generally equal to the external diameter of catheter tube 324 so that the inner surface of the sheath 312 slidingly engages the delivery catheter 310.

The sheath may be made of any suitable biocompatible material, for example PTFE, nylon, or polyethylene. The sheath may optionally comprise a flat wire coil (not shown) to provide the sheath with additional flexibility and kink-resistance. U.S. Patent No. 5,380,304 and U.S. Published Patent Application No. 2001/0034514 A1, propose various reinforced sheaths and methods of making the same that may be used in the present invention.

As shown in FIG 8, the prosthesis 320 may comprise a stent graft having a plurality of self-expanding stents 332, as described above. The stents 332 cause the prosthesis 320 to expand during its release from the device. The stents 332 may cover and/or may be at least partially covered by a graft material. The prosthesis 320 also may include an exposed self-expanding zigzag stent 334 for anchoring the prosthesis 320 in the body lumen. The zigzag stent 334 may comprise barbs 336 that extend from the stent. When the zigzag stent 334 is released, the barbs 336 engage the surrounding lumen.

The prosthesis 320 is retained in a radially reduced configuration between the delivery catheter 310 and the sheath 312. The sheath 312 is slidably disposed over the prosthesis 320 and the delivery catheter 310 in a proximal and a distal direction. The sheath 312 may be slid proximally with respect to the delivery catheter 310 and the prosthesis 320 to expose the prosthesis. To deploy the prosthesis 320, the operator slides the sheath 312 proximally while applying distal pressure to the delivery catheter 310 via catheter tube 324. Catheter tube 324 pushes the prosthesis 320 distally via the annular abutment surface 323 while the sheath 312 slides proximally in relation thereto. As the sheath 312 slides proximally, the catheter tube 324 pushes the prosthesis 320 distally from the receiving portion 316 and into the body lumen.

The delivery and deployment device may further comprise a haemostatic sealing device 319 for controlling blood loss between the delivery catheter 310 and the sheath 312 during a procedure. The haemostatic sealing device 319 may also include a side tube 330 that facilitates the introduction of medical reagents between the delivery catheter 310 and the sheath 312.

The delivery and deployment device may optionally include deployment control mechanisms 339, 340, as shown in FIG 8. Proximal control mechanism 339 releasably retains the proximal end of the prosthesis 320 and distal control mechanism 340 releasably retains the distal end of the prosthesis 320. Proximal control mechanism 339 may comprise a trigger wire (not shown) that releasably couples the proximal end of the prosthesis 320 to the delivery catheter 310. Likewise, the distal control mechanisms 340 may comprise a trigger wire (not shown) that releasably couples the distal end of the prosthesis 320 to the delivery catheter 310. The trigger wires extend proximally to the external manipulation section 303 where they are coupled to trigger release devices 343, 344. Trigger release devices 343, 344 are configured selectively to decouple the proximal and distal ends of the prosthesis from the delivery catheter 310, respectively. Various prosthesis retention devices, configurations, and methods of use are disclosed in PCT Publication No. WO 98/53761.

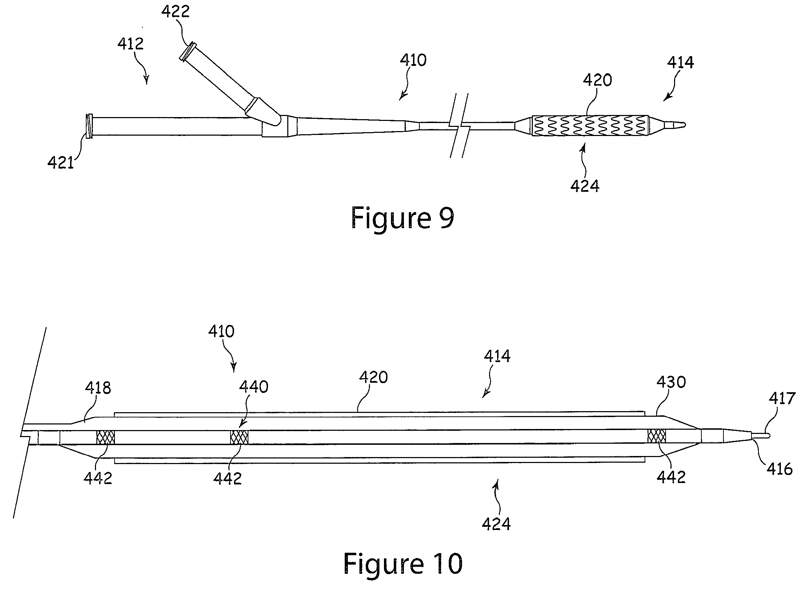

FIGS 9 and 10 illustrate another device for delivering and deploying a partially or entirely balloon-expandable prosthesis 420 into a body lumen. As shown in FIG 9, the device comprises a balloon catheter 410 having a proximal portion 412 and a distal portion 414. The distal portion 414 is configured to be inserted into a patient to deliver the prosthesis 420 to a damaged or diseased body lumen. The proximal portion 412 remains outside of the patient and is manipulated by the operator during a procedure to deliver and deploy the prosthesis.

FIG 10 shows a partial cross-sectional view of a distal portion 414 of the balloon catheter 410. The balloon catheter 410 comprises a guide wire lumen 416 and a balloon inflation lumen 418. The guidewire lumen 416 is adapted to receive a guidewire 417 during a procedure. The balloon inflation lumen 418 is adapted to deliver pressurized fluid to expand the balloon. As shown in FIG 9, the proximal portion 412 of the delivery device comprises a guidewire port 421 for inserting the guidewire 417 into the guidewire lumen 416 and a balloon inflation port 422 for introducing pressurised fluid into the inflation lumen 418.

The balloon catheter further includes a stent loading area 424 and may include a stent positional indicator system 440 located on a distal portion 414 of the catheter 410. The stent-loading area 424 comprises an inflatable balloon 430. The prosthesis 420 is loaded onto the deflated balloon 430 in a compressed configuration. The positional indicator system 440 includes one or more positional indicators 442 that may be used to indicate various portions of the prosthesis, for example, a fenestration. Indicators 442 may include, for example, radiopaque marker bands.

In operation, the prosthesis 420 is positioned about the unexpanded balloon 430 on the catheter and crimped thereto so that desired portions of the prosthesis 420 align with corresponding components of the positional indicator system 440. If the positional indicators 442 are disposed on the distal portion of the catheter 410, within the balloon, the balloon 430 may comprise a generally transparent material so that the marker system 440 may easily be viewed during loading.

The balloon catheter 410 may comprise any balloon configuration suitable for expanding the prosthesis 420. In addition, the balloon catheter 410 may be configured to expand one or both ends of the graft, for example, to engage another module. For example, the balloon may comprise multiple balloons or balloon portions along its length, where each portion has a distinct expansion characteristic. U.S. Patent Application Publication Nos. 2005/02222668, 2005/0171598, and 2005/0149166, disclose delivery systems for intraluminal prostheses having single and multiple balloons.

### INTRALUMINAL BYPASS METHODS

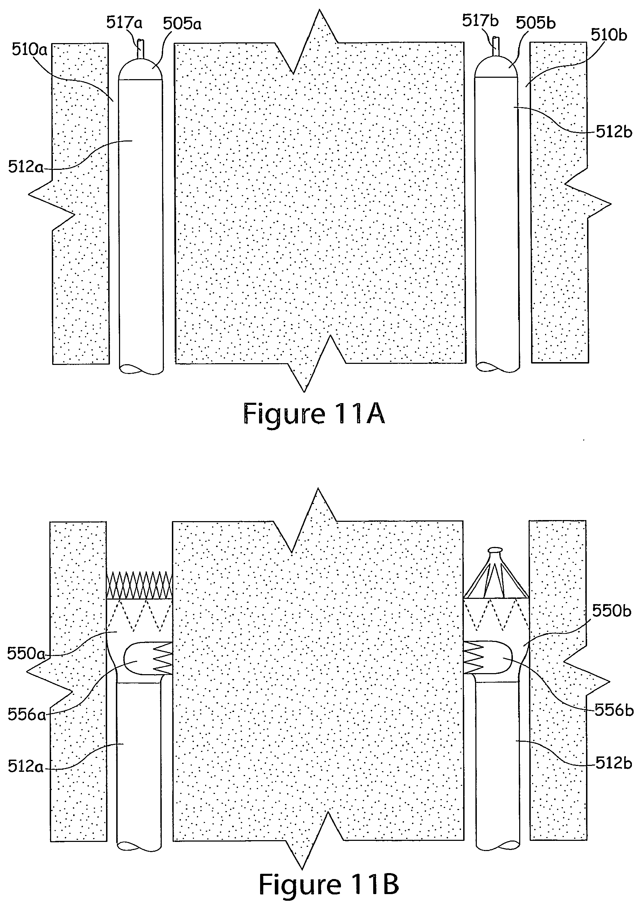

FIGs 11A-F illustrate various stages of an exemplary intraluminal bypass method in which an intraluminal prosthesis is delivered and deployed in a vessel system comprising a first vessel segment 510a and a second vessel segment 510b.

The bypass method does not form part of the invention as claimed.

Such a method may be performed, for example, to provide a bypass between vessel segments in two distinct vessels, as shown in FIG 4. Alternatively, a method may be performed to provide a bypass between vessel segments of a single vessel, as shown in FIG 5.

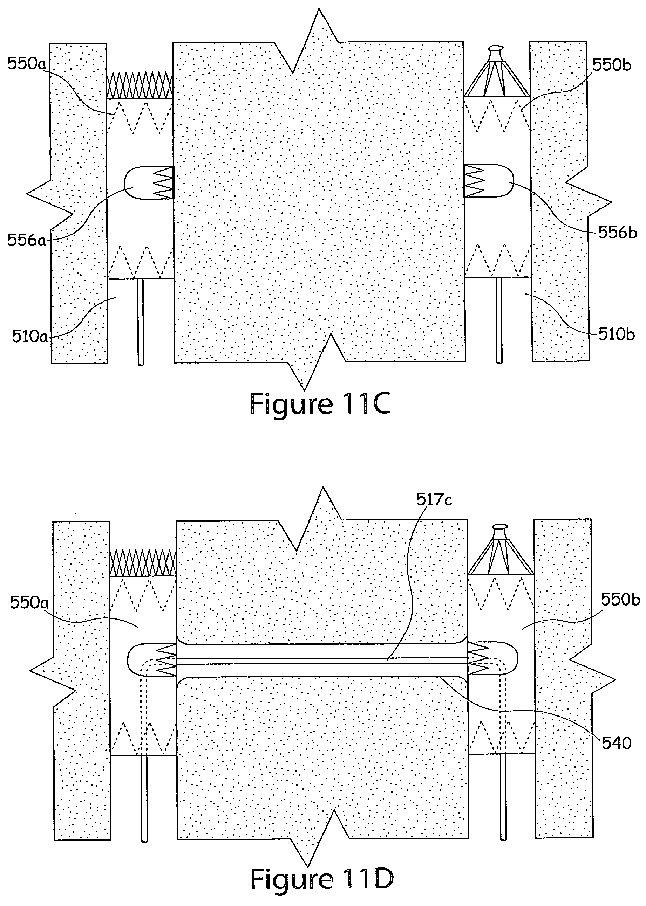

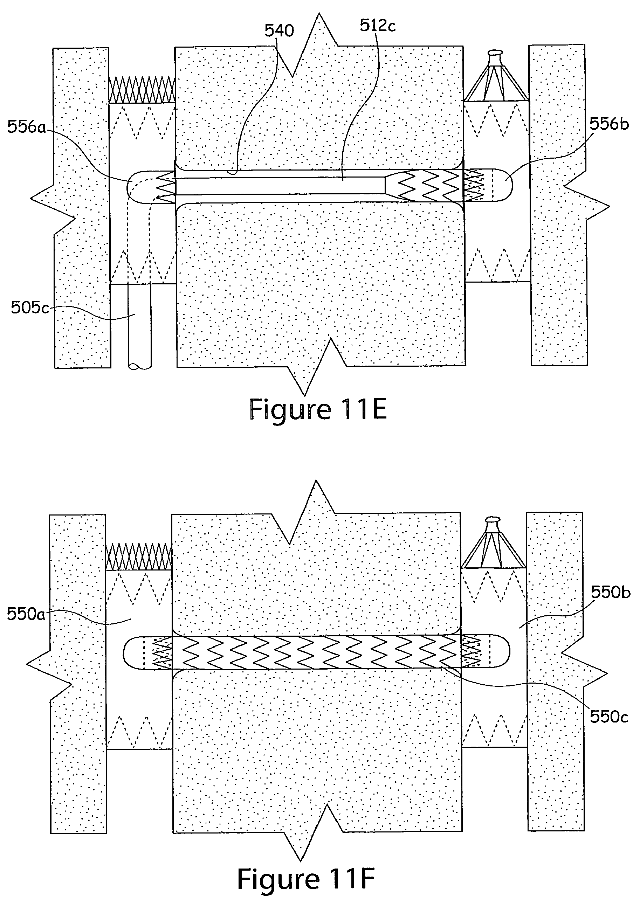

In FIG 11A, a first delivery and deployment device 505a is disposed adjacent a first vessel segment 510a and carries a first prosthetic module 550a, and a second delivery and deployment device 505b is disposed adjacent a second vessel segment 510b and carries a second prosthetic module 550b. Although FIG 11 depicts delivery and deployment of devices 505a, 505b in parallel (*i.e.,* at the same time), it is also contemplated that the devices could be delivered in series (*i.e*., one after the other).

The delivery and deployment devices 505a, 505b may be delivered over one or more guidewires 517a, 517b that have been placed using radiographic techniques that are generally known in the art. At this stage, each prosthetic module 550a, 550b is fully retained in a respective delivery and deployment device in a radially-constrained configuration by a sheath 512a, 512b.

Once the modules 550a, 550b are properly positioned, the delivery and deployment devices 505a, 505b are ready for deployment. The operator may retract each of the sheaths 512a, 512b, by pulling the sheaths in a proximal direction over the respective module. As each sheath retracts, the prosthetic modules 550a, 550b are exposed and are allowed to expand into the vessel segments. If the modules are self-expanding, they may expand simply by removing the sheath, as shown in FIG 11 b.

FIG 11B shows the prosthetic modules 550a, 550b in a partially deployed state. At this point, the operator may adjust the axial and radial position of each module to ensure that the fenestrations 556a, 556b are properly positioned and oriented.

FIG 11C, shows the prosthetic modules 550a, 550b in a fully deployed state and the delivery and deployment devices have been removed. At this point, additional modules (not shown) may be delivered and deployed within one or both of the vessel segments to extend the modules within the vessel segments.

Next, one or more tunnelling devices (not shown), such as a balloon cutting device, may be provided for creating a pathway or channel between the two prosthetic modules 550a, 550b. In one example, a single tunnelling device may be provided and inserted through the first vessel segment 510a and the fenestration 556a of the first module 550a. In this example, the device cuts through the wall of the first vessel segment 510a, tunnels through the interluminal space towards the second vessel segment 510b, and cuts through the wall of the second vessel segment adjacent the second fenestration 556b. In another example, two tunnelling devices may be provided, one in each vessel segment 510a, 510b. In this example, the tunnelling devices may be inserted through the fenestrations 556a, 556b and tunnel through the vessel walls and interluminal space towards a common central point. To facilitate the tunnelling step, the tunnelling devices may comprise sensing means for sensing the fenestrations. For example, the tunnelling device could comprise a magnet and the fenestrations could comprise a suitable magnetic material.

In FIG 11D, a channel 540 has been created between the first and second prosthetic modules 550a, 550b and a guide wire 517c is delivered within the channel 540 between the modules.

Next, as shown in FIG 11E, delivery and deployment device 505c (carrying prosthetic module 550c) is delivered into the channel 540 via one of the prosthetic modules. For example, the device 505c may be inserted through the first prosthetic module 550a and the first fenestration 556a. The prosthetic module 550c is positioned so that it can be attached to the first and second prosthetic modules 550a, 550b via first and second fenestrations 556a, 556b. Once prosthetic module 550c is properly positioned, the operator retracts sheath 512c, thereby exposing prosthetic module 550c and allowing it to expand within the channel 540. The distal end of prosthetic module 550c expands and sealingly engages fenestration 556b.

In FIG 11F, the prosthetic module 550c is shown in a fully deployed state and the delivery and deployment device 505c has been removed. The proximal end of prosthetic module 550c expands and sealingly engages fenestration 556a.

### VESSEL REPAIR KITS

Various tools are available to the physician to facilitate intraluminal delivery and deployment of a prosthesis. For example, imaging techniques such as fluoroscopy, CT, and MRI are available and may be used prior to or during the procedure to help the physician understand the patient's anatomy and to anticipate challenges and improve the procedural outcome. In many situations, it would be advantageous for the surgeon to be able to practise delivering and deploying the prosthesis before, or even during, the actual procedure.

Accordingly, a deployment model may be provided to the physician that comprises, for example, a physical model of a patient's anatomy. The model preferably corresponds generally with, and substantially replicates, relevant vessels and the diseased condition of the patient. In addition, an intraluminal prosthesis may be provided for delivery and deployment within the deployment model. The intraluminal prosthesis is preferably substantially identical to an intraluminal prosthesis that may be provided for delivery and deployment within the patient. The physician may deliver and deploy the intraluminal prosthesis within the deployment model prior to, or even during, a patient procedure.

In one example, a kit may be provided for an intraluminal procedure and comprise a deployment model, a first intraluminal prosthesis, and a second intraluminal prosthesis. The kit does not form part of the invention as claimed.

The deployment model comprises one or more vessels corresponding generally with a patient's vessels. The first and second intraluminal prostheses are preferably substantially identical. For example, the size, shape, and configuration of the first prosthesis may be generally the same as the size, shape, and configuration of the second prosthesis.

In one example, the first prosthesis is provided for delivery and deployment within the patient, whereas the second prosthesis is provided for delivery and deployment within the model. Accordingly, although the second prosthesis is substantially identical to the first intraluminal prosthesis, the second prosthesis may comprise insubstantial differences over the first prosthesis. For example, the first prosthesis may be sterilised for use with the patient, whereas the second prosthesis may not be sterilised. The second prosthesis may be produced, for example, using less expensive materials and methods that do not substantially alter the overall structure and function of the device. For example, if the prosthesis is a stent graft that comprises one or more stents sutured to a graft material, the second stent graft may be provided using a less expensive graft, stent, and/or suture materials or methods than those used for the first graft.

The model may be produced using conventional moulding or casting fabrication techniques, such as injection, compression, transfer, and extrusion moulding, thermoforming, and die casting. In some examples, the model may be fabricated using a rapid prototyping technique, such as stereolithography, lamininated object manufacturing, selective laser sintering, fused deposition modeling, and 3-D printing. The model may include any suitable material including, but not limited to, metals, such as stainless steel and aluminium, and plastics, such as acrylonitrile butadiene styrene, polycarbonate, polyester, polyamide, polystyrene, polyphenyl sulfone, and suitable thermoplastic elastomers. The material may be rigid and/or flexible, depending on the application. In some examples, a model may be provided and comprise regions of relative flexibility and regions of relative rigidity that replicate the unique characteristics of the patient's vasculature.

A separate kit may be provided to the physician for each patient so that the physician can practise the patient's procedure prior to the actual procedure, or even during the procedure, for example, if unexpected difficulties were to be encountered. Each model may be fabricated based on imaging data that is provided for a respective patient. The data may be converted to a suitable CAD format, such as .STL file format and input into rapid prototyping software to produce a vessel model.

Throughout this specification various indications have been given as to preferred and alternative embodiments of the invention. However, it should be understood that the invention is not limited to any one of these. It is therefore intended that the foregoing detailed description be regarded as illustrative rather than limiting, and that it be understood that it is the appended claims that are intended to define the scope of this invention.

## Claims

1. An intraluminal bypass prosthesis (250;550) including:
a first prosthetic module (60;250b,550b) having a first end (66;252b), a second end (68;254b), and a fenestration (70;256b,556b) disposed between the first and second ends of the first prosthetic module, wherein an end (66;252b) of the first prosthetic module is at least partially occluded to prevent fluid flow through that end;
a second prosthetic module (30 in figures 2C and 2D;250a;550a) having a first end, a second end, and a fenestration (50;256a;556a) disposed between the first and second ends of the second prosthetic module; and
a third prosthetic module (30 in figures 2A, 2E, 2F;250c;550c) having a first end that is sealingly engageable with the first prosthetic module within its fenestration, a second end that is sealingly engageable with the second prosthetic module within its fenestration, and a lumen between the first and second ends of the third prosthetic module for providing fluid communication between the first and second prosthetic modules, wherein the first and second ends of the third prosthetic module include an anchor (40) that allows it sealingly to engage the first prosthetic module or the second prosthetic module, the anchor including one or more stents or stent portions (34a, 34c) and/or one or more hooks and/or barbs (42);
wherein the first prosthetic module is suitable to be placed in a first vessel segment, the second prosthetic module is suitable to be placed in a second vessel segment, where the first vessel segment and the second vessel segment include portions of the same vessel.

2. A prosthesis as claimed in claim 1, wherein an end of the second prosthetic module is at least partially occluded (66) to prevent fluid flow through that end.

3. A prosthesis as claimed in claim 1 or 2, wherein the first prosthetic module includes a graft material that traverses and closes the lumen at the end of the first prosthetic module.

4. A prosthesis as claimed in claim 1, 2 or 3, wherein at least one of the first, second, and third prosthetic modules is a stent graft and includes one or more stents (34, 36, 38; 64).

5. A prosthesis as claimed in any preceding claim, wherein at least one of the fenestrations includes a branch lumen (52) extending from the prosthetic module.

6. A prosthesis as claimed in any preceding claim, wherein the third prosthetic module includes a balloon-expandable stent disposed at the first and second ends and one or more self-expanding stents disposed intermediate the first and second ends.

## Patentansprüche

1. Intraluminale Bypass-Prothese (250; 550), die Folgendes aufweist:
ein erstes Prothesemodul (60; 250b, 550b) mit einem ersten Ende (66; 252b), einem zweiten Ende (68; 254b) und einer Fensterung (70; 256b, 556b), die zwischen dem ersten und dem zweiten Ende des ersten Prothesemoduls angeordnet ist, wobei ein Ende (66; 252b) des ersten Prothesemoduls mindestens teilweise verschlossen ist, um Fluidströmung durch dieses Ende zu verhindern,
ein zweites Prothesemodul (30 in Figuren 2C und 2D; 250a; 550a) mit einem ersten Ende, einem zweiten Ende und einer Fensterung (50; 256a; 556a), die zwischen dem ersten und dem zweiten Ende des zweiten Prothesemoduls angeordnet ist, und
ein drittes Prothesemodul (30 in Figuren 2A, 2E, 2F; 250c; 550c) mit einem ersten Ende, das dichtend mit dem ersten Prothesemodul in seiner Fensterung in Eingriff bringbar ist, einem zweiten Ende, das dichtend mit dem zweiten Prothesemodul in seiner Fensterung in Eingriff bringbar ist, und einem Lumen zwischen dem ersten und dem zweiten Ende des dritten Prothesemoduls, um Fluidkommunikation zwischen dem ersten und dem zweiten Prothesemodul bereitzustellen, wobei das erste und das zweite Ende des dritten Prothesemoduls einen Anker (40) aufweisen, der gestattet, dass es das erste Prothesemodul oder das zweite Prothesemodul dichtend in Eingriff nimmt, wobei der Anker einen oder mehrere Stents oder Stentabschnitte (34a, 34c) und/oder einen oder mehrere Haken und/oder Widerhaken (42) aufweist,
wobei sich das erste Prothesemodul für ein Platzieren in einem ersten Gefäßsegment eignet, und sich das zweite Prothesemodul für ein Platzieren in einem zweiten Gefäßsegment eignet, wobei das erste Gefäßsegment und das zweite Gefäßsegment Abschnitte desselben Gefäßes aufweisen.

2. Prothese nach Anspruch 1, wobei ein Ende des zweiten Prothesemoduls mindestens teilweise verschlossen (66) ist, um Fluidströmung durch dieses Ende zu verhindern.

3. Prothese nach Anspruch 1 oder 2, wobei das erste Prothesemodul ein Graft-Material aufweist, das das Lumen durchquert und dieses am Ende des ersten Prothesemoduls verschließt.

4. Prothese nach Anspruch 1, 2 oder 3, wobei das erste und/oder das zweite und/oder das dritte Prothesemodul ein Stentgraft ist und einen oder mehrere Stents (34, 36, 38; 64) aufweist.

5. Prothese nach einem der vorhergehenden Ansprüche, wobei mindestens eine der Fensterungen ein Zweiglumen (52) aufweist, das sich vom Prothesemodul erstreckt.

6. Prothese nach einem der vorhergehenden Ansprüche, wobei das dritte Prothesemodul einen ballon-expandierbaren Stent, der am ersten und am zweiten Ende angeordnet ist, und einen oder mehrere selbstexpandierende Stents, die zwischen dem ersten und dem zweiten Ende angeordnet sind, aufweist.

## Revendications

1. Prothèse de dérivation intraluminale (250 ; 550) comprenant :
un premier module prothétique (60 ; 250b ; 550b) comportant une première extrémité (66 ; 252b), une seconde extrémité (68 ; 254b), et un orifice (70 ; 256b ; 556b) disposé entre les première et seconde extrémités du premier module prothétique, une extrémité (66 ; 252b) du premier module prothétique étant au moins partiellement occluse de façon à empêcher un écoulement de fluide par cette extrémité ;
un deuxième module prothétique (30 dans les figures 2C et 2D ; 250a ; 550a) comportant une première extrémité, une seconde extrémité et un orifice (50 ; 256a ; 556a) disposé entre les première et seconde extrémités du deuxième module prothétique ; et
un troisième module prothétique (30 dans les figures 2A, 2E, 2F ; 250c ; 550c) comportant une première extrémité qui peut être accouplée de manière étanche avec le premier module prothétique à l'intérieur de son orifice, une seconde extrémité qui peut être accouplée de manière étanche avec le deuxième module prothétique à l'intérieur de son orifice, et une lumière entre les première et seconde extrémités du troisième module prothétique assurant la communication fluidique entre les premier et deuxième modules prothétiques, les première et seconde extrémités du troisième module prothétique comprenant un élément de fixation (40) qui lui permet de s'accoupler de manière étanche avec le premier module prothétique ou le deuxième module prothétique, l'élément de fixation comprenant un(e) ou plusieurs stents ou parties de stent (34a, 34c) et/ou un(e) ou plusieurs crochets et/ou pointes (42) ;
le premier module prothétique étant conçu pour pouvoir être placé dans un premier segment de vaisseau, le deuxième module prothétique étant conçu pour pouvoir être placé dans un deuxième segment de vaisseau, le premier segment de vaisseau et le deuxième segment de vaisseau comprenant des parties du même vaisseau.

2. Prothèse selon la revendication 1, dans laquelle une extrémité du deuxième module prothétique est au moins partiellement occluse (66) de façon à empêcher un écoulement de fluide par cette extrémité.

3. Prothèse selon la revendication 1 ou 2, dans laquelle le premier module prothétique comprend un matériau de greffe qui traverse et ferme la lumière au niveau de l'extrémité du premier module prothétique.

4. Prothèse selon la revendication 1, 2 ou 3, dans laquelle au moins l'un des premier, deuxième et troisième modules prothétiques est une endoprothèse couverte et comprend un ou plusieurs stents (34, 36, 38 ; 64).

5. Prothèse selon l'une quelconque des revendications précédentes, dans laquelle au moins l'un des orifices comprend une lumière formant ramification (52) s'étendant à partir du module prothétique.

6. Prothèse selon l'une quelconque des revendications précédentes, dans laquelle le troisième module prothétique comprend un stent expansible par ballonnet disposé au niveau des première et seconde extrémités et un ou plusieurs stents auto-expansibles disposés entre les première et seconde extrémités.
